Europäisches Patentamt

European Patent Office

Office européen des brevets

(19)

(11) Publication number: **0 027 303**

**B1**

(12) **EUROPEAN PATENT SPECIFICATION**

(45) Date of publication of patent specification: **27.07.83**

(51) Int. Cl.³: **A 41 B  13/02**

(21) Application number: **80301988.4**

(22) Date of filing: **12.06.80**

(54) **Process and apparatus for attaching elastic strips in an elastic leg disposable diaper, and diapers produced by said process and apparatus.**

(30) Priority: **16.10.79 US  85372**
**16.10.79 US  85382**
**08.02.80 US  115280**

(43) Date of publication of application:
**22.04.81 Bulletin 81/16**

(45) Publication of the grant of the patent:
**27.07.83 Bulletin 83/30**

(84) Designated Contracting States:
**AT BE CH DE FR GB IT LI LU NL SE**

(56) References cited:
**EP - A - 0 000 969**
**EP - A - 0 017 770**
**FR - A - 2 413 048**
**FR - A - 2 415 433**
**US - A - 3 860 003**
**US - A - 4 081 301**

(73) Proprietor: **Riegel Textile Corporation**
**Post Office Box 290 Grand Central Station 290**
**Madison Avenue**
**New York, New York 10017 (US)**

(72) Inventor: **Teed, Richard Kellogg**
**110 Rutledge Road**
**Greenwood South Carolina (US)**
Inventor: **Gore, Graves Thomas**
**2310 Pine Log Road**
**Aiken South Carolina (US)**

(74) Representative: **Warren, Keith Stanley et al,**
**BARON & WARREN 18 South End Kensington**
**London W8 5BU (GB)**

Courier Press, Leamington Spa, England

Process and apparatus for attaching elastic strips in an elastic leg disposable diaper and diapers produced by said process and apparatus

This invention relates to an improved elastic leg disposable diaper having elastic strips extending and being secured to the diaper continuously along the full length of the outside longitudinal edges of the diaper, wherein the elastic strips are secured in the crotch area of the diaper in stretched elastically-contractible condition forming gathered and extendible side portions in the crotch area for elastic conformance of such side portions with the legs of the wearer, and a process and apparatus for attaching such elastic strips in the diapers.

Over the past decade, disposable diapers, which include fluid-absorbent interior pads positioned between fluid-permeable top cover sheets and fluid-impermeable bottom cover sheets, have become increasingly popular. These disposable diapers are fabricated and sold in various shapes, sizes and constructions.

More recently, disposable diapers known in the trade as "elastic leg" disposable diapers have been introduced and are gaining popularity. These disposable diapers are preferably of generally hour-glass configuration including a central crotch-fitting area and outer waist-fitting areas and have elastic strips secured along the outside longitudinal edges of the crotch area of the disposable diapers for forming extendible side portions along the crotch area of the diaper for elastic compliance to the legs of the wearer, such as has heretofore been available with waterproof panties utilized in diapering infants. Examples of commercial forms of such elastic leg diapers may be seen in The Procter & Gamble Company's U.S. Patent 3,860,003 and Kimberly-Clark Corporation's U.S. Patent 4,050,462.

Although the elastic leg disposable diapers commercialized under each of these two prior art patents are apparently successful in the marketplace, the diapers of these two prior art patents suffer from certain disadvantages relating to the manner in which the elastic strips are secured within the longitudinal edges of the diaper which result in structural deficiences and an expensive and complicated procedure of inserting such elastic strips.

Although the Procter & Gamble U.S. Patent 3,860,003 does not fully and clearly disclose the process utilized in inserting the elastic strips within the longitudinal edges of the disposable diaper, its subsequent U.S. Patent 4,081,301 clearly discloses such process. As may be seen in this Procter & Gamble U.S. Patent 4,081,301 and in the Kimberly-Clark U.S. Patent 4,050,462, both of these elastic leg disposable diaper products involve the insertion of the elastic leg disposable diaper products involve the insertion of the elastic strips within the longitudinal edges of the disposable diaper being manufactured in serially-interconnected form by a process in which adhesive is intermittently applied to spaced-apart portions or lengths of continuous strips of elastic and the elastic strips are continuously stretched and secured within the longitudinal edges of the disposable diapers so that the portions of the elastic strips having adhesive thereon are secured within the crotch area of the diaper and the lengths of the elastic strips without adhesive thereon are positioned within the longitudinal edges of the outer waist areas of the diaper. With this construction, when the disposable diapers are cut apart into individual diapers and the elastic strips are cut, the portions of the elastic strips which are unsecured within the waist-fitting portions of the diapers will snap within the diaper leaving loose ends therein and the portion of lengths of the elastic strips secured within the crotch area of the diaper will gather such areas forming extendible side portions.

As a result of the above manufacturing process, the snapping in of the loose or unsecured ends of the elastic strips at the outer waist-fitting areas of the diaper may result in damage to the diaper and leave open passages through the transverse edges of the diaper which may be susceptible to moisture leaks, etc. Also, this prior manufacturing process requires complicated and expensive equipment, such as that disclosed in the above-identified Procter & Gamble U.S. patent 4,081,301.

An alternative approach is disclosed in French Patent Specification FR - A - 2 415 433. This French patent discloses a disposable diaper in which an elastic film material is attached along the longitudinal edges of a diaper, and either (1) the film is thermoplastic and is applied in a prestretched condition along the entire longitudinal edges of the diaper and is rendered non-elastic through heat in the waist-fitting areas, or (2) the film is heat-shrinkable and is applied in a non-stretched condition along the entire longitudinal edges of the diaper and is rendered elastic in the crotch-fitting areas through the application of heat. This approach, necessitates the use of special elastic film materials, and the process and apparatus involved are relatively complex and costly.

It is an object of this invention to provide an elastic leg disposable diaper and process and apparatus for attaching elastic strips along the longitudinal edges of such a diaper which overcome the problems associated with prior elastic leg diaper products and the processes and apparatus for attaching the elastic strips therein.

According to one aspect of the present invention, there is provided a process of securing elastic strips within longitudinal edges of elastic leg disposable diapers having central crotch-fitting areas and outer waist-fitting areas, the elastic strips extending across the crotch-fitting areas between the waist-fitting areas to form gathered and extendible side portions in the crotch areas of the diapers during manufacture of such diapers, the process including the steps of feeding continuous elastic strips from a supply into desired positions in contact within the longitudinal edges of the diapers, while effecting adhesive securement therebetween along substantially the entire lengths of the edges, and characterised by alternately stretching and substantially relaxing predetermined lengths of the elastic strips during said feeding step, while adhering the stretched lengths of the elastic strips to the crotch areas and the more relaxed lengths of the elastic strips to the waist areas susbstantially continuously along the entire longitudinal edges of the diapers, so that the stretched lengths of the elastic strips contract and form gathered and extendible side portions in the crotch areas of the diapers.

According to a further aspect of the present invention, there is provided apparatus for carrying out the process just defined, for attaching elastic strips in elastic leg disposable diapers having gathered and extendible side portions in crotch areas during the manufacture and movement of such diapers through an assembly machine, including means for feeding continuous elastic strips into desired positions along the longitudinal edges of the diapers for adhesive attachment along substantially the entire edges, and characterised by means for alternately stretching and relaxing predetermined lengths of the elastic strips during the feeding thereof for attaching the stretched lengths to the crotch areas and the more relaxed lengths to outer waist areas substantially continuously along the longitudinal edges of the diapers.

According to another aspect of the present invention there is provided an elastic leg disposable diaper produced by the process or apparatus just defined.

In one embodiment, the stretching and relaxing may be obtained by alternate feeding of the elastic strips at a first speed substantially equal to the speed of movement of the diapers during manufacture of such diapers for maintaining the elastic strips in substantially relaxed condition and then at a second speed less than the speed of movement of the diapers during manufacture of such diapers for stretching the elastic strips.

In another embodiment, the elastic strips are fed at a predetermined constant rate and the alternate stretching and relaxing of predetermined lengths of the continuous elastic strips is obtained, as follows:

The elastic strips are nipped during the feeding thereof at spaced-apart points to define a predetermined distance of travel of the elastic strips between the nipping points. One of the nipping points is positioned at the point of adhesive attachment of the elastic strips in the diapers. The elastic strips are engaged between the nipping points and alternately moved to other positions to vary the predetermined distance of travel of the elastic strips for alternately stretchng and relaxing lengths of the elastic strips.

Inasmuch as feeding of elastic strips necessarily places some tension and causes some stretching and cannot be accomplished in a totally tensionless or relaxed condition and since the elastic strips are being fed at a constant rate from the supply which must be less than the length of the diapers since portions thereof are being attached in stretched condition and since the elastic strips are in effect underfed for the relaxed lengths and overfed for the stretched lengths, some provision must be provided for compensating for these conditions. Accordingly, the elastic strips are first moved quickly to a position for increasing the distance of travel of the elastic strips between nipping points for stretching lengths of the elastic strips and then incrementally further moved for further increasing the distance of travel of the elastic strips for a predetermined period of time to compensate and maintain the stretching of the lengths of elastic strips while the stretched lengths are fed into position for adhesive attachment in the diapers. Thereafter, the distance of travel of the elastic strips is quickly decreased for relaxing lengths of the elastic strips and then the distance of travel of the elastic strips is incrementally further decreased for a predetermined period of time to compensate and maintain the relaxing of the lengths of elastic strips while they are being fed into position in the diapers.

In order that the invention may be more readily understood, reference will now be made to the accompanying drawings, in which:

Figure 1 is a perspective view of an elastic leg disposale diaper embodying this invention in flat condition with the side portions of the crotch area in an extended position;

Figure 2 is a cross-sectional view taken through the crotch area of the diaper and generally along the line 2—2 of Figure 1;

Figure 3 is a cross-sectional view taken through one of the outer waist areas of the diaper and generally along the line 3—3 of Figure 1;

Figure 4 is a top perspective view of the diaper of Figure 1 with the side portions of the crotch area in relaxed, gathered and extendible position;

Figure 5 is a cross-sectional view taken through one of the longitudinal edges of the relaxed diaper of Figure 4 and generally along the line 5—5 of Figure 4;

Figure 6 is a schematic perspective view of apparatus for manufacturing elastic leg disposable diapers utilizing a first embodiment of apparatus for attaching elastic strips in the diapers in accordance with this invention;

Figure 7 is an enlarged schematic view of a portion of the apparatus of Figure 6 and taken from a different angle and further illustrating the details of the first embodiment of the apparatus for inserting elastic strips into the diapers in accordance with this invention;

Figure 8 is a schematic perspective view of apparatus for manufacturing elastic leg disposable diapers utilizing a second embodiment of apparatus for attaching elastic strips in the diapers in accordance with this invention;

Figure 9 is an elevational view, partly in section, of the second embodiment of the apparatus for attaching elastic strips into the diapers and taken generally in the direction of arrow 9 of Figure 8;

Figure 10 is a schematic perspective view of the second embodiment of the apparatus for attaching elastic strips into the diapers with some devices omitted and with the bottom cover sheet of the diaper omitted for clarity of illustration;

Figure 11 is a schematic perspective view of the second embodiment of apparatus for attaching elastic strips into the diapers and taken from the opposite side of the view of Figure 10 and generally in the direction of arrow 11 in Figure 10 and with some devices omitted and with the bottom cover sheet of the diaper omitted for clarity of illustration;

Figure 12 is a schematic of a timed control mechanism for the second embodiment of apparatus for attaching elastic strips into the diapers.

Figure 13 is an enlarged side elevational schematic view of a portion of the apparatus of Figure 8 and taken generally in the direction of arrow 13 of Figure 8;

Figure 14 is a partial, elevational view, taken generally along the line 14—14 of Figure 13;

Figure 15 is a diagram illustrating the motion of the devices for stretching and relaxing the elastic strips in accordance with the second embodiment of apparatus illustrated in Figure 8; and

Figures 16—19 are side elevational schematic views illustrating a portion of the apparatus shown in Figure 13 with the devices for stretching and relaxing the elastic strips in various positions during operation of the apparatus in accordance with the diagram of Figure 15.

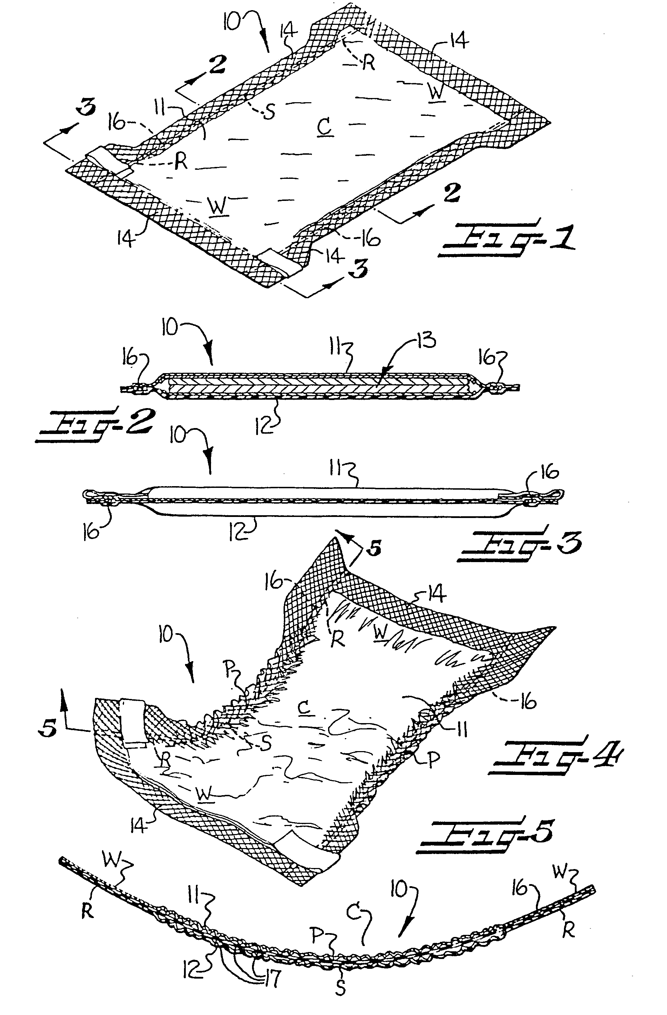

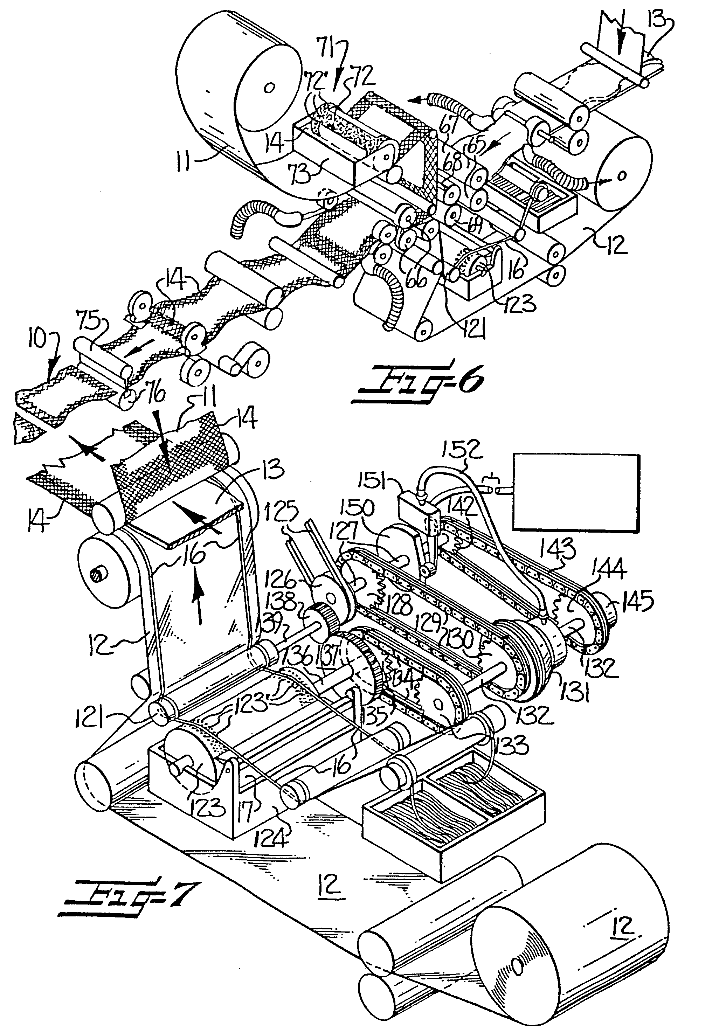

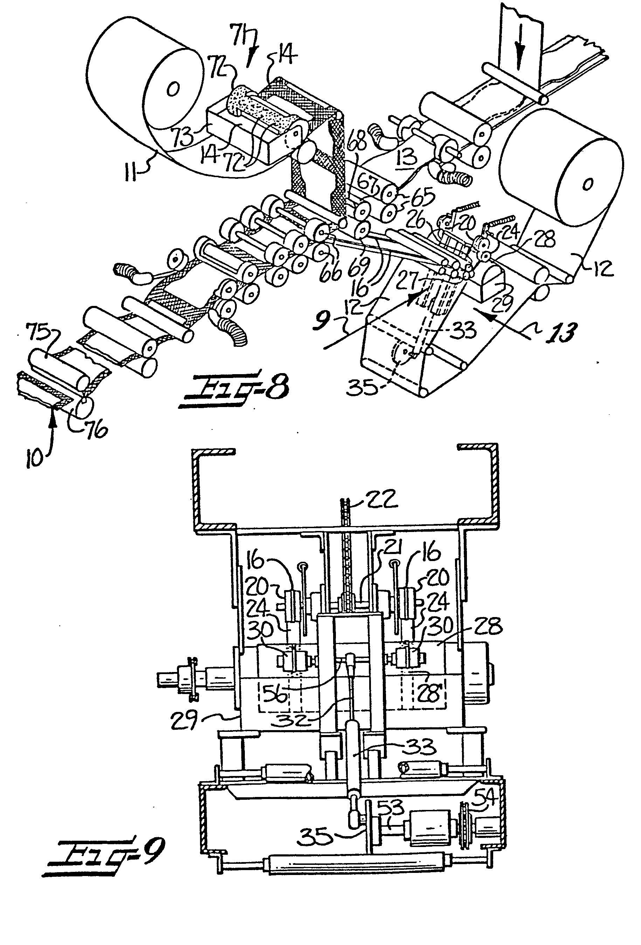

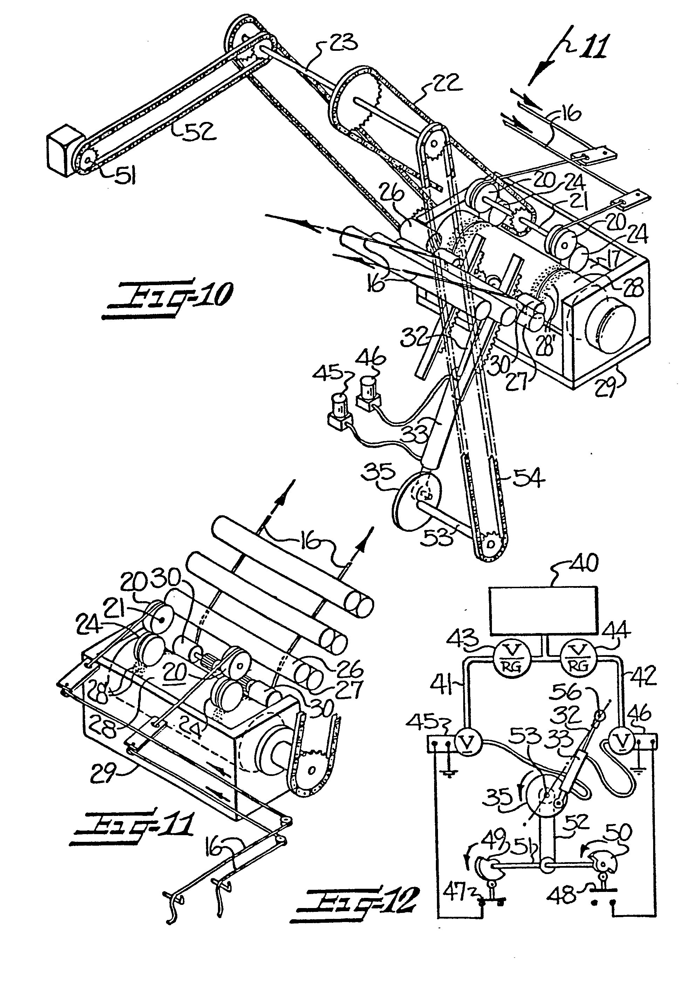

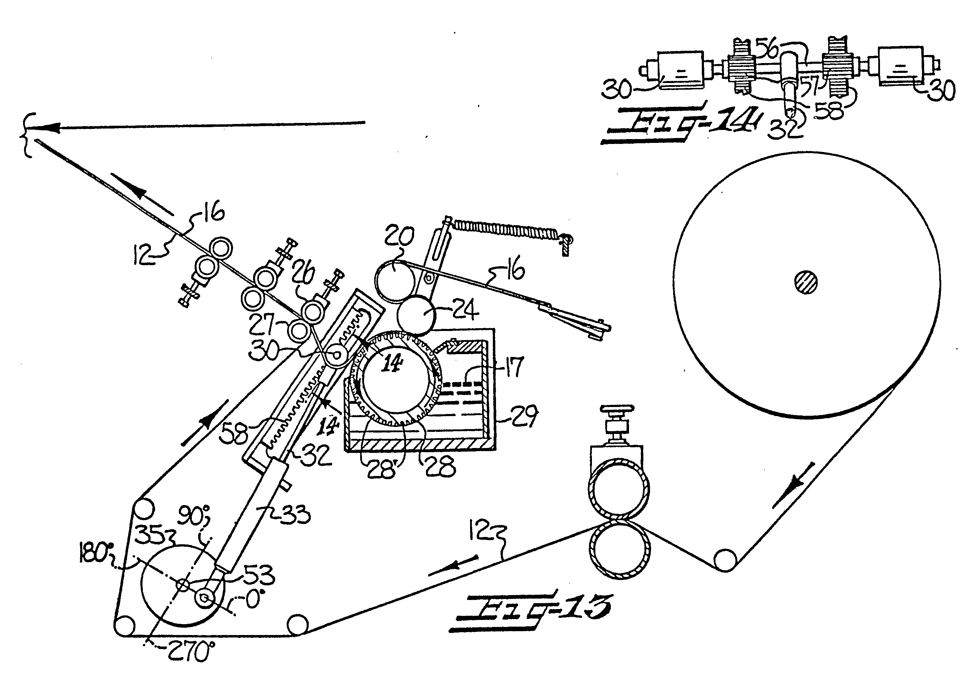

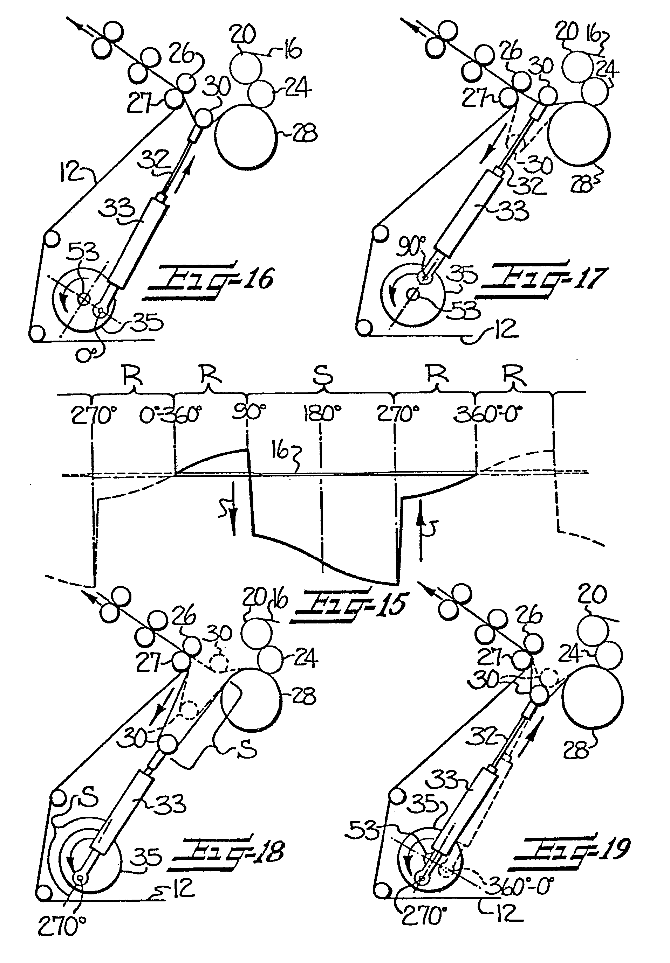

In the drawings, Figures 1—5 illustrate the improved elastic leg disposable diaper, and Figures 6 and 7 illustrate a first embodiment of apparatus and Figures 8—19 illustrate a second embodiment of apparatus for attaching elastic strips into the elastic leg disposable diaper and for practicing the process of this invention.

Referring firstly to Figures 1—5, the improved elastic leg disposable diaper, generally indicated at 10, is preferably of generally hour-glass configuration and has a central crotch-fitting area C and outer waist-fitting areas W designed to be placed around the crotch and waist of the wearer when the diaper 10 is placed on the wearer in conventional manner.

The diaper 10 further includes a fluid-permeable top cover sheet 11, which may be of any suitable construction conventionally utilized in disposable diaper and other absorbent article constructions for being positioned in contact with the wearer of the disposable diaper 10 for receiving and passing therethrough body fluids of the wearer. The diaper 10 further includes a fluid-impermeable bottom cover sheet 12, which may be constructed of plastic or other suitable material conventionally utilized in such disposable diaper and other disposable absorbent article constructions for being positioned away from the wearer for preventing the body fluids of the weaver from passing out of the disposable diaper 10. Positioned between the top cover sheet 11 and the bottom cover sheet 12 is a fluid-absorbent interior pad 13 for absorbing body fluids of the wearer. As illustrated in Figure 2, this interior absorbent pad 13 may include two layers or may be a single layer or any other suitable construction conventionally utilized in such disposable diaper constructions.

The disposable diaper 10 is secured along its longitudinal and transverse edges by suitable adhesive or other attachment means 14 which secures the top sheet 11 to the bottom sheet 12 and generally provides an envelope around the interior pad which is unsecured at its longitudinal edges and in the central crotch area C to either the top cover sheet or bottom cover sheet to allow the pad to conform to the shape of the diaper when placed in position on the wearer.

The diaper 10 further includes elastic strips 16 extending and being secured by adhesive 17, see Figure 5, to the diaper 10 continuously along the full length of the outside longitudinal edges of the diaper, as indicated in Figures 1, 4 and 5. These elastic strips, as will be explained in detail below, are secured in the crotch area C in stretched elastically-contractible condition forming gathered and extendible side portions P in the crotch area C, as shown in Figures 4 and 5, for elastic compliance to the legs of the wearer. Preferably, the elastic strips 16 are secured in the waist areas W in less elastically-contractible or relaxed condition so as to provide less or no gathering of the side portions of the waist areas W, as indicated in Figures 4 and 5.

As may be clearly seen in Figures 1 and 2, the interior absorbent pad 13 extends to close proximity with and within approximately 18 mm (3/4 inch) of the elastic strips 16 on both of the longitudinal sides of the diaper 10 to provide fluid absorbency in the elastically-contractible side portions P of the diaper 10.

Considering now the processes and apparatus embodying this invention, reference may be had to Figures 6—7 and Figures 8—19 which show schematically portions of apparatus suitable for use in producing the elastic leg disposable diapers 10 and first and second embodiments, respectively, of apparatus for attaching elastic strips 16 into the diapers 10. Full details of mechanical connections, drives, etc. are not illustrated and will not be described herein for purposes of clarity. The apparatus illustrated in Figures 6 and 13 for manufacturing or assembling disposable diapers 10 are similar and differ only in the respective first and second embodiments of apparatus for attaching elastic strips 16. Accordingly, like reference numerals will be utilized for like diaper assembly apparatus in both of these illustrations. Many details of the construction and operation of the apparatus for assembling such diapers are not shown in these illustrations and are not shown in these illustrations and are not necessary for a full understanding of the present invention; however, such details may be seein in co-pending United States application Serial No. 046,114, filed June 6, 1979 and prior United States Patents Re. 28,139 and 3,984,272, all of which are assigned to us.

Referring now to the apparatus illustrated in Figures 6 and 8 for assembling disposable diapers, interconnected multi-layer absorbent pads 13 are shown being fed through pairs of driven feed rolls 65, 66 between which is provided a pad severing means in the form or a rotating cutting roll 67 having a knife blade 68 thereon and an anvil roll 69 which operate to intermittently cut the interconnected multi-layer pads 13 transversely for separation of the interconnected pads 13. The driven feed rolls 66 are driven somewhat faster than the driven feed rolls 65 to effect an overfeeding of the severed interior pads 13 for spacing the pads apart in the further fabrication of the disposable diapers 10.

Immediately prior to the feed rolls 66, the top cover sheet 11 is fed from a suitable source of supply to feed rolls 66 and through a glue applicator 71 which applies the glue or abrasive 14 of a suitable type in predetermined pattern across the cover sheet 11 at spaced locations and along the longitudinal edges of the top cover sheet 11 for purposes of gluing the transverse and longitudinal edges of the ultimately fabricated disposable diaper 10 together. Glue appliator 71 may comprise any suitable glue applicator for the above purpose and as illustrated herein may include a glue applicator roll 72 having indentations or cups 72' therein which collectively are of the predetermined configuration of the areas for receiving adhesive 14. The applicator roll 72 may pass through an adhesive supply tank 73 for containing a supply of the adhesive 14.

Simultaneously with the above, the bottom cover sheet 12 is fed from a source of supply to feed rolls 66 and into superimposed position under the spaced-apart interior pads 13 and the top cover sheet 11 so that all of these components are in superimposed position. The top cover sheet 11 and the bottom cover sheet 12 are pressed into contact with each other by the feed rolls 66 and subsequent rolls and are adhesively secured together along longitudinal edges and transversely between the separated and spaced apart absorbent pads to form serially-interconnected disposable diapers 10 which are continuously fed through the remaining portions of the apparatus by suitable feeding means.

These serially-interconnected diapers 10 are fed through a series of mechanisms, not shown, and are ultimately fed between cutter roll 75 and anvil roll 76 which cut the serially-interconnected diapers 10 transversely between interior pads to form individual diapers 10.

Cooperating with the above-described apparatus for manufacturing or assembling disposable diapers 10, apparatus in accordance with the invention is provided for attaching elastic strips 16 during the manufacture of such disposable diapers 10. This apparatus includes means for alternately stretching and relaxing predetermined lengths of elastic strips 16 for adhesive securement of the stretched elastic lengths $S$ to the crotch area $C$ and the more relaxed lengths $R$ to the outer waist areas $W$ continuously along the longitudinal edges of the diaper 10, as may be seen particularly in Figures 1, 4 and 5.

Referring now the first embodiment, illustrated in Figures 6 and 7, of this apparatus for attaching elastic strips 16 during the manufacture or assembly of such disposable diapers 10, this first embodiment includes means for feeding continuous elastic strips 16 with adhesive thereon along the entire length thereof into desired positions for adhesive attachment in the diapers. This feeding means may include driven feed roll 121 mounted to receive elastic strips 16 from a suitable source of supply and feed the continuous elastic strips into engagement with the bottom cover sheet 12 and into engagement with the guide rolls of the apparatus for assembling the disposable diapers 10 and receiving the top cover sheet 11, bottom cover sheet 12 and interior pads 13 in superimposed position.

In the case of the apparatus illustrated herein, the elastic strips 16 are fed into contact with and over an adhesive applicator roll 123 which includes cups or indentations 123' therein for picking up adhesive 17 as it passes through a supply tank 124 containing such adhesive 17. The applicator roll 123 is adapted to apply adhesive continuously along the entire face of the elastic strips 16 as they are fed by feed roll 121. The adhesive 17 is desirably of a fast-acting type since the elastic strips are being secured in both the stretched and relaxed conditions and the adhesive must set up as soon as the elastic strips are moved into contact with one of the component of the disposable diaper 10, such as the bottom cover sheet 12 in the case of the apparatus illustrated in Figures 6 and 7.

For driving the feed roll 121 to effect alternate stretching and relaxing of the elastic strips 16, selectively variable drive means are provided.

As illustrated in Figure 7, this selectively variable drive means may include a first selectively operable drive means comprising a belt 125 driven from a suitable motor or other drive mechanism which passes around pulley 126 to rotate shaft 127 which carries sprocket gear 128 thereon. Sprocket gear 128 receives chain 129 which in turn drives sprocket 130 which is connected through a pneumatic clutch 131 to shaft 132 which drives sprocket 133, chain 134 and sprocket 135. Sprocket 135 in turn drives shaft 136 which is connected to and rotates glue applicator roll 123. The shaft 136 carries gear 137 which meshes with gear 138 carried on the drive shaft 139 of feed roll 121 for driving of feed roll 121.

The above-described drive constitutes a first drive means for the elastic strip feeding means, i.e. feed roll 121, and is constructed when actuated through pneumatic clutch 131 to feed the elastic strips 16 at substantially the same speed of travel as the components 11, 12, 13 of the disposable diapers are travelling through the disposable diaper manufacturing or assembly apparatus, described above, so that the elastic strips 16 will be secured within the disposable diapers 10 in relaxed condition.

A second selectively operable drive means is provided which includes sprocket 142 carried on shaft 127 and driving chain 143 which in turn drives sprocket 144 which drives shaft 132 through slip clutch 145 and thus drives adhesive applicator roll 123 and feed roll 121 through the above-described mechanism. This last described second drive means is a slower speed drive means and when operable will drive the feed roll 121 at a speed less than the speed of travel of the components 11, 12, 13 of the disposable diapers through the disposable diaper manufacturing apparatus, so that the elastic strips 16 will be stretched since the feed roll 121 is in effect underfeeding the elastic strips 16 into engagement with the diapers 10. This stretching will occur between the feed roll 121 and the point in which the elastic strips 16 are brought into adhesive securing engagement with the bottom cover sheet 12.

For controlling operation of the above-described selectively operable first and second drive means, there is provided a timed control means which is operatively connected with each of the drive means for alternately actuating each of the drive means in correlation with the speed of movement of the diapers 10 through the assembly machine and the lengths of the longitudinal edges of the crotch area *C* and the waist areas *W* to which the alternate lengths of stretched and relaxed lengths *S*, *R* of the elastic tapes 16 are to be secured.

This timed control means may comprise a cam 150 mounted on and rotated by drive shaft 127 and which includes a cut-out portion and a lobe portion thereon designed to open and close valve 151 contained within an air conduit 152 extending from any suitable source of air supply to the pneumatic clutch 131, so that, when the valve 151 is opened by the cam 150, the air clutch 131 will be actuated to effect operation of the first drive means which drives the feed roll 121 at a faster speed of travel. When the cam 150 closes valve 151 and thus deactuates pneumatic clutch 131, the feed roll 121 will be driven through the second drive means through the slip clutch 145. It will be understood by those with ordinary skill in the art that the slip clutch 145 will slip when the faster drive through pneumatic clutch 131 is operating.

The cam 150 may be easily designed by one with ordinary skill in the art to open and close valve 151 in accordance with the lengths of the longitudinal edges of the respective crotch area and waist areas of the serially-interconnected diapers 10 so that the alternately stretched and relaxed lengths of the elastic strips 16 may be applied in desired position within the diapers 10.

Referring next to the second embodiment, illustrated in Figures 8—19, of apparatus for attaching elastic strips 16 during the manufacture or assembly of disposable diapers 10, this second embodiment includes means for feeding the continuous elastic strips 16 from a suitable source of supply (not shown) at a predetermined constant rate into desired position for adhesive attachment in the diapers. As illustrated in Figures 10 and 11, the feeding means may include driven feed rolls 20 commonly mounted on a driven shaft 21 for receiving the elastic strips 16 from a suitable source of supply. From the feed rolls 20, the elastic strips 16 are fed into engagement with the bottom cover sheet 12 for immediate adhesive attachment thereto and are then fed by feed rolls 66 into position with the remaining components of the disposable diaper, as illustrated in Figure 8. The drive shaft 21 is driven by a suitable chain drive 22 from a shaft 23 which may, in turn, be driven by suitable drive mechanisms (not shown) from the diaper assembly machine at a constant rate.

The means for alternately stretching and relaxing predetermined lengths of the elastic strips 16 further includes two spaced-apart nipping means cooperating with the feeding means and engaging the elastic strips 16 during the feeding thereof and defining a predetermined distance of travel of the elastic strips 16 between the nipping means. In the embodiment of the apparatus illustrated herein, the two spaced-apart nipping means comprise the feeding rolls 20 and cooperating guide rolls 24 which are suitably mounted for pivotal biased engagement with the feed rolls 20, as shown in Figure 13, and the elastic strips 16 pass in generally an S-shaped path around the feed rolls 20 and the guide rolls 24 and are nipped therebetween to form the first nipping means. The second nipping means consists of a pair of opposed rolls 26, 27 which are mounted for nipping engagement with each other in any suitable manner and are spaced from the first nipping means 20, 24 to form a predetermined distance of travel of the elastic strips therebetween, as may be seen in Figure 13. The second nipping means 26, 27 also receives the bottom cover sheet 12 so that the elastic strips 16 are adhesively attached to this diaper component at this point.

From the nipping means formed by the rolls 20, 24, the elastic strips 16 pass over and into contact with a driven adhesive appliator roll 28 which includes cups or indentations 28' therein for picking up adhesive 17 as it passes through a supply tank 29 containing such adhesive 17. The applicator roll 28 is adapted to apply adhesive continuously along one of the faces of each of the elastic strips 16 as they are fed past the driven applicator roll 28. The driven applicator roll 28 may also be in nipping engagement with the guide rolls 24 and constitute the first nipping means. The adhesive 17 is desirably of a fast-acting type since the elastic strips are being secured in both the stretched and relaxed condition and the adhesive must set up as soon as the elastic strips are moved into contact with one of the components of the disposable diaper 10, such as the bottom cover sheet 12 in the case of the apparatus illustrated herein.

The means for alternately stretching and relaxing predetermined lengths of the elastic strips 16 further comprises movable means, such as rolls 30, positioned for engaging the elastic strips 16 as they are fed between the nipping means 20, 24 or 24, 48 and 26, 27 and for alternately moving to other positions (as shown in Figures 16—19) to vary the distance of travel of the elastic strips 16 between such nipping means for alternately stretching and relaxing lengths of the elastic strips 16.

Means are connected with the elastic strip engaging roll means 30 for moving same and comprise a piston and cylinder 32, 33 connected to the roll means 30 and mounted for alternate quick movement to and from a contracted position (dotted line position of Figure 17 and solid line position of Figure 18) and an extended position (solid line positions of Figures 16, 17 and 19) for alternately moving the elastic strip engaging roll means 30 quickly to a position for increasing the distance of travel of the elastic strips 16 between the nipping means for stretching the lengths $S$ of elastic strips 16 and then quickly to a position decreasing the distance of travel of the elastic strips 16 for relaxing lengths $R$ of the elastic strips 16.

There is further provided a crank means including rotating crank disc 35 and a crank arm which is formed by the piston and cylinder 32, 33 and has one end eccentrically pivotally mounted on the rotating crank disc and the other end carrying the elastic strip engaging roll means 30 for translating the rotary motion of the crank disc 35 to incremental reciprocation of the elastic strip engaging roll means 30 for alternately moving the elastic strip engaging roll means 30 to positions incrementally further increasing the distance of travel of the elastic strips 16 after the increase thereof by the piston and cylinder 32, 33 to maintain the stretching of the lengths $S$ of elastic strips 16 during the feeding thereof (as is illustrated during rotation of the crank disc from the position of Figure 17 to the position of Figure 18) and then to positions incrementally further decreasing the distance of travel of the elastic strips 16 after the decrease thereof by the piston and cylinder means 32, 33 to maintain the relaxing of the lengths $R$ of elastic strips 16 during the feeding thereof (as is illustrated during the rotation of the crank disc 35 from the position shown in Figure 18 to the position shown in Figure 17).

The means for alternately stretching and relaxing predetermined lengths of the elastic strips 16 further comprises timed control means (a schematic of which is illustrated in Figure 12) connected with and controlling operation of the piston and cylinder 32, 33 and the crank means 35, 32 and 33, which form the means for moving the elastic strip engaging roll means 30, in correlation with the speed of movement of the diapers 10 through the assembly machine and the lengths of the longitudinal edges of the crotch area $C$ and the waist areas $W$ to which alternate stretched and relaxed lengths $S, R$ of the elastic strips 16 are to be attached.

As shown in Figure 12, this timed control means may comprise a source of pressurized air 40 which provides pressurized air to a pair of conduits 41, 42 through pressure regulating valves 43, 44 and to electrically operated valves 45, 46. From the valves 45, 46 the air conduits 41, 42 extend to opposite sides of the cylinder 33 for respective actuation of the piston and cylinder 32, 33 for quick movement to the extended position and the contracted position. The valves 45, 46 are controlled for alternate actuation by electrical switches 47, 48 which are controlled by cams 49, 50 mounted on a common shaft 51 driven by a chain or belt drive 52 from a driven shaft 53 which carries and drives the crank disc 35. The shaft 53 is connected by chain or belt drive 54 to driven shaft 23 which is driven from drive mechanisms of the diaper assembly machine in timed correlation with the drives for feeding the diapers 10 through the assembly machine during their fabrication.

For stabilizing movement of the strip engaging rolls 30, they are mounted on a common shaft 56 attached to the piston 32 and which carries pinions 57 positioned for up and down movement in racks 58.

For operation of the above-described apparatus for attaching elastic strips 16 in elastic leg disposable diapers 10 during the manufacture of such diapers 10 in the assembly machine, the following operation and process may be utilized.

A typical disposable diaper 10 is approximately 44 cm (17.8 inches) in length and the central crotch area C would be approximately 22 cm (8.9 inches) in length and the outer waist areas 11 cm (4.45 inches) in length for a combined length of 22 cm (8.9 inches). Therefore, in order to attach relaxed lengths R and stretched lengths S of the continuous elastic strips 16 alternately to the waist areas W and the crotch areas C of the interconnected diapers 10, a relaxed length R of elastic strips 16 of approximately 22 cm (8.9 inches) must be fed by the apparatus of this invention into adhesive engagement in proper position along the longitudinal edges of the interconnected diapers 10 and then a stretched length S of the elastic strips 16 of approximately 22 cm (8.9 inches) must be fed into position in the diaper 10 which requires stretching of the stretched length S to approximately twice its original length which will result in the feeding of approximately 11 cm (4.45 inches) of unstretched elastic strips 16 for attachment in the crotch area C.

This stretching and relaxing is accomplished by actuation of the piston and cylinder mechanism 32, 33 from its extended position (Figure 16) which feeds the elastic strips 16 in substantially relaxed condition into desired positions for attachment along abutting waist areas W of two interconnected diapers to the contracted position (dotted line position of Figure 17) which doubles the length of travel of the elastic strips 16 between nipping means and stretches the elastic strips 16 so that the same effective lengths of elastic strips 16 are fed into position along the crotch area of the diaper 10, while only one-half that length or 11 cm (4.45 inches) of elastic strips are fed from the unstretched supply.

However, inasmuch as the elastic strips 16 are being fed at a constant rate from the supply, which can be no more than 11 cm (4.45 inches) for the stretched lengths S and 22 cm (8.9 inches) for the relaxed lengths R for a total of 33 cm (13.35 inches) of unstretched elastic strips per 44 cm (17.8 inches) diaper, and because the feeding of elastic strips necessarily places some tension and causes some stretching and cannot be accomplished in a totally tensionless or relaxed condition, some means must be provided for compensating for the tension or stretch caused by normal feeding and for the constant feeding rate which is in effect underfeeding for the relaxed lengths R and overfeeding for the stretched lengths S. This compensation is provided by the motion of the crank means which incrementally further increases the distance of travel of the elastic strips S for a predetermined period of time to maintain stretching of the lengths S of the elastic strips 16 after stretching thereof by the piston and cylinder 32, 33 and while they are being fed into position in the diapers 10 and then incrementally further decreases the distance of travel of the elastic strips 16 for a predetermined period of time after they have been relaxed by operation of the piston and cylinder 32, 33 to maintain the relaxing of the lengths R of elastic strips 16 while they are being fed into position in the diapers 10.

Referring now to the diagram of Figure 15 and Figures 16—19 which show relative positions of the piston and cylinder 32, 33 and the crank means 35 and 32, 33 during various positions of operation, it may be seen in Figure 16 that when the crank means is in its 0° position which is the position in which the elastic strips 16 are being attached at the start of a waist area W of one of the interconnected diapers 10, the elastic strips 16 are in a relatively relaxed condition. To maintain this relaxed condition during the feeding of the strips 16, the crank mechanism rotates around to its 90° position of Figure 17 which in effect incrementally decreases the distance of travel of the elastic strips 16 between nipping points to further relax the elastic strips 16 for the purposes discussed above and as indicated in the diagram of Figure 15. At this position, the piston and cylinder mechanism is operated by the opening of valve 46 through the closing of switch 48 by cam 50 to move the piston and cylinder 32, 33 to its contracted position as shown in dotted lines in Figure 17. This immediately doubles the length of the path of travel of the elastic strips 16 between nipping points for stretching the lengths S of elastic strips 16. This stretch is maintained for a predetermined period of time during rotation of the crank disc 35 to its 270° position as shown in Figure 18 during which the predetermined distance of travel of the elastic strips 16 is incrementally increased by effective downward movement of the roll 30 to the position shown in Figure 18 and as indicated in the diagram of Figure 15. At this point, the piston and cylinder mechanism, 32, 33 is operated by the opening of valve 45 by the switch 47 being closed by cam 49 to move the piston and cylinder 32, 33 to its extended position, as indicated in dotted lines in Figure 18 and in solid lines in Figure 19, for quickly relaxing the elastic strips 16 as indicated in the diagram of Figure 15. This relaxing is maintained during the next 90° of rotation of the cam disc 35 by incrementally further decreasing the predetermined distance of travel of the elastic strips 16 between nipping points, as indicated in the diagram of Figure 15, through the moving of the roll 30 upward as indicated in dotted lines in Figure 19. The cycle of operation of alternately stretching and relaxing the elastic strips 16 for application to one disposable diaper 10 has now been completed and the cycle is repeated.

Thus, this invention has provided two embodiments of apparatus capable of performing the broad process of this invention of securing elastic strips 16 within longitudinal edges of elastic leg disposable diapers 10 having central crotch-fitting areas C and outer waist-fitting areas W to form gathered and extendible side portions P in the crotch area C of the diapers 10 during the manufacture of such diapers 10. The broad process of this invention includes the steps of feeding continuous elastic strips 16 from a supply into desired position in contact with the longitudinal edges of the diapers 10, while effecting adhesive securement therebetween along the entire length of the edges, and alternately stretching and relaxing predetermined lengths S, R of the continuous elastic strips 16 during the feeding step for adhering the stretched lengths S of the continuous elastic strips 16 to the crotch area C and the more relaxed lengths R of the continuous elastic strips 16 to the waist areas W continuously along the longitudinal edges of the diapers 10, so that the stretched lengths S of the elastic strips will contract and form gathered and extendible side portions P in the crotch area C of the diapers 10.

Preferably, the process also involves applying adhesive continuosly to the elastic strips 16 during the feeding step along the entire length of the elastic strips 16.

A more specific process of this invention is performed wherein the step of alternately stretching and relaxing predetermined lengths S, R of the continuous elastic strips 16 comprises feeding the elastic strips 16 at a first speed substantially equal to the speed of movement of the diapers during manufacture of such diapers for maintaining lengths R of the elastic strips 16 in a substantially relaxed condition and at a second speed less than the speed of movement of the diapers 10 during manufacture of such diapers for stretching lengths S of the elastic strips 16.

Another more specific process of this invention involves feeding the elastic strips 16 at a predetermined constant rate and the step of alternately stretching and relaxing predetermined lengths S, R of the continuous elastic strips 16 comprises nipping the elastic strips 16 during the feeding thereof at spaced-apart points to define a predetermined distance of travel of the elastic strips 16 between the nipping points, quickly increasing the distance of travel of the elastic strips 16 for stretching lengths S of the elastic strips 16 and incrementally further increasing the distance of travel of the elastic strips 16 for a predetermined period of time to maintain the stretching of the lengths S of elastic strips 16 while they are being fed into position in the diapers 10, and then quickly decreasing the distance of travel of the elastic strips 16 for relaxing lengths R of the elastic strips 16 and incrementally further decreasing the distance of travel of the elastic strips 16 for the predetermined period of time to maintain the relaxing of the lengths R of elastic strips 16 while they are being fed into position in the diapers 10.

As will be readily understood by those with skill in the art, it is difficult if not impossible to obtain complete relaxation of the elastic strips 16 during any feeding operation and the term "relaxed", as used herein and as results from a practical operation of the apparatus of this invention and the practice of the processes herein, is relative and is intended to cover the feeding and application of elastic strips 16 in a "more relaxed" condition when compared with the stretched condition. Accordingly, the elastic strips 16 will probably always be somewhat or slightly tensioned or stretched even in the waist areas W of the disposable diaper 10.

## Claims

1. A process of securing elastic strips (16) within longitudinal edges of elastic leg disposable diapers (10) having central crotch-fitting areas (C) and outer waist-fitting areas (W), the elastic strips (16) extending across the crotch-fitting areas (C) between the waist-fitting areas (W) to form gathered and extendible side portions (P) in the crotch areas of the diapers during manufacture of such diapers, the process including the steps of feeding continuous elastic strips (16) from a supply into desired positions in contact within the longitudinal edges of the diapers (10), while effecting adhesive securement therebetween along substantially the entire lengths of the edges, and characterised by alternately stretching and substantially relaxing predetermined lengths of the elastic strips during said feeding step, while adhering the stretched lengths (S) of the elastic strips to the crotch areas (C) and the more relaxed lengths (R) of the elastic strips to the waist areas (W) substantially continuously along the entire longitudinal edges of the diapers, so that the stretched lengths (S) of the elastic strips contract and form gathered and extendible side portions (P) in the crotch areas of the diapers.

2. A process as claimed in claim 1, characterised by the step of applying the adhesive substantially continuously along the length of the elastic strips (16).

3. A process as claimed in claim 1 or 2, characterised in that the step of alternately stretching and relaxing predetermined lengths of the elastic strips (16) comprises feeding the elastic strips at a first speed substantially equal to the speed of movement of the diapers (10) during manufacture of such diapers for maintaining the elastic strips in a substantially relaxed condition and at a second speed less than the speed of movement of the diapers during manufacture of such diapers for stretching the elastic strips.

4. A process as claimed in claim 1 or 2, characterised in that the step of feeding the elastic strips (16) comprises feeding the strips at a predetermined constant rate, and the step of alternately stretching and relaxing predetermined lengths of the elastic strips comprises nipping the elastic strips during the feeding thereof at spaced-apart points to define a predetermined distance of travel of the elastic strips between the nipping points, quickly increasing the distance of travel of the elastic strips for stretching lengths (S) of the elastic strips and incrementally further increasing the distance of travel of the elastic strips for a predetermined period of time to maintain the stretching of the lengths of the elastic strips while they are being fed into position in the diapers (10), and then quickly decreasing the distance of travel of the elastic strips for relaxing lengths (R) of the elastic strips and incrementally further decreasing the distance of travel of the elastic strips for a predetermined period of time to maintain the relaxing of the lengths of elastic strips while they are being fed into position in the diapers.

5. Apparatus for carrying out the process claimed in claim 1, for attaching elastic strips (16) in elastic leg disposable diapers (10) having gathered and extendible side portions (P) in crotch areas (C) during the manufacture and movement of such diapers through an assembly machine, including means (20, 121) for feeding continuous elastic strips (16) into desired positions along the longitudinal edges of the diapers (10) for adhesive attachment along substantially the entire edges, and characterised by means for alternately stretching and relaxing predetermined lengths of the elastic strips during the feeding thereof for attaching the stretched lengths (S) to the crotch areas (C) and the more relaxed lengths (R) to outer waist areas (W) substantially continuously along the longitudinal edges of the diapers.

6. Apparatus as claimed in claim 5, characterised in that the means for alternately stretching and relaxing predetermined lengths of the elastic strips (16) comprises selectively variable drive means (125—145) connected with the elastic strip feeding means (121) for driving said feeding means at a first speed substantially equal to the speed of movement of the diapers (10) in the assembly machine for maintaining the elastic strips in relaxed condition and at a second speed less than the speed of movement of the diapers in the assembly machine for stretching the elastic strips, and control means (150, 151) operatively connected with the drive means for alternately actuating said drive means for the first and second speeds of feed of the elastic strips for alternately actuating said drive means for the first and second speeds of feed of the elastic strips for alternately stretching and relaxing predetermined lengths of the elastic strips.

7. Apparatus as claimed in claim 6, characterized in that the selectively variable drive means comprises first drive means (128—131) connected with the elastic strip feeding means (121) and the control means (150, 151) for being selectively operated for driving said elastic strip feeding means at the first speed, and second drive means (142—145) operatively connected with the elastic strip feeding means and the control means for being selectively operated for driving said elastic strip feeding means at the second speed.

8. Apparatus as claimed in claim 6 or 7, characterized in that the control means includes timing means (150) for alternately operating said first and second drive means in correlation with the speed of movement of the diapers through the assembly machine and the lengths of the longitudinal edges of the crotch areas and the waist areas to which alternate lengths of stretched and relaxed portions of the elastic strips are to be adhered.

9. Apparatus as claimed in claim 5, characterized in that the means for feeding the elastic strips (16) comprises means (20) for feeding the elastic strips at a predetermined constant rate and the means for alternately stretching and relaxing predetermined lengths of the elastic strips comprises two spaced-apart nipping means (20, 24 and 26, 27) cooperating with said feeding means and engaging the elastic strips during feeding thereof and defining a predetermined distance of travel of the elastic strips between said nipping means, one of said nipping means (26, 27) being positioned at the point of adhesive attachment of the elastic strips in the diapers (10), movable means (30) positioned for engaging the elastic strips as they are fed between said nipping means and for alternately moving to other positions to vary the distance of travel of the elastic strips for alternately stretching and relaxing lengths of the elastic strips, and means (32, 33, 35) connected with said movable means (30) for moving same for quickly increasing the distance of travel of the elastic strips for stretching lengths of the elastic strips and incrementally further increasing the distance of travel of the elastic strips for a predetermined period of time to maintain the stretching of the lengths of the elastic strips while they are being fed into position in the diapers, and then quickly decreasing the distance of travel of the elastic strips for relaxing lengths of the elastic strips and incrementally further decreasing the distance of travel of the elastic strips for a predetermined period of time to maintain the relaxing of the lengths of elastic strips while they are being fed into position in the diapers.

10. Apparatus as claimed in claim 9, characterized in that the means for alternately stretching and relaxing predetermined lengths of the elastic strips includes timed control means (40—52) connected with the means (32, 33, 35) for moving the movable means (30) for operating such means in timed correlation with the speed of movement of the diapers through the assembly machine and the lengths of the longitudinal edges of the crotch areas and the waist areas to which alternate lengths of stretched and relaxed portions of the elastic tapes are to be attached.

11. Apparatus as claimed in claim 9 or 10, characterized in that the means for moving the movable means (30) comprises piston and cylinder means (32, 33) connected to said movable means and mounted for quick movement to and from a contracted position and an extended position for moving said movable means to the positions for stretching and relaxing the elastic strips (16), and crank means including a rotating crank disc (35) and a crank arm formed by said piston and cylinder means (32, 33) and having one end thereof eccentrically pivotally mounted on said rotating crank disc and the other end thereof carrying said movable means for translating the rotary motion of said crank disc to incremental reciprocation of said movable means for maintaining the stretching and relaxing of the elastic strips.

12. Apparatus as claimed in any one of claims 5 to 10, characterised by means (28, 123) cooperating with the feeding means (20, 21) for applying adhesive continuously along the elastic strips (16) as they are fed by said feeding means for adhesive attachment of the elastic strips in the diapers.

13. An elastic leg disposable diaper (10) produced by the process claimed in any of claims 1 to 4, or by the apparatus claimed in any of claims 5 to 12.

**Patentansprüche**

1. Verfahren zum Befestigen elastischer Streifen (16) innerhalb der Längsränder von Wegwerf-Windeln (10) mit elastischem Beinabschluß, die mittlere in der Schrittregion anliegende Zonen (C) (Schritt-Zonen) und äußere in der Taillenregion anliegende Zonen (W) (Hüft-Zonen) aufweisen, wobei sich die elastischen Streifen (16) über die Schritt-Zonen (C) zwischen den Hüft-Zonen (W) erstrecken und so geraffte und dehnbare Seitenteile (P) in den Schritt-Zonen der Windeln während der Herstellung derselben bilden, bei welchem Verfahren endlose elastische Streifen (16) von einem Vorrat in gewünschte Positionen in Berührung mit den Längsrändern der Windeln (10) zugeführt werden und hierbei eine gegenseitige Klebeverbindung über praktisch die gesamte Länge der Ränder bewirkt wird, dadurch gekennzeichnet, daß man vorbestimmte Längen der elastischen Streifen während des Schritts des Zuführens abwechselnd streckt und im wesentlichen entspannt, wobei man die ge-

streckten Längen (S) der elastischen Streifen an die Schritt-Zonen (C) und die mehr entspannten Längen (R) der elastischen Streifen an die Hüft-Zonen (W) im wesentlichen durchgehend längs der gesamten Längsränder der Windeln anklebt, so daß sich die gestreckten Längen (S) der elastischen Streifen zusammenziehen und geraffte und dehnbare Seitenteile (P) in den Schritt-Zonen der Windeln bilden.

2. Verfahren nach Anspruch 1, dadurch gekennzeichnet, daß man den Klebstoff im wesentlichen durchgehend über die Länge der elastischen Streifen (16) aufbringt.

3. Verfahren nach Anspruch 1 oder 2, dadurch gekennzeichnet, daß man die dem Schritt des abwechselnden Streckens und Entspannens vorbestimmter Längen der elastischen Streifen (16) diese mit einer ersten Geschwindigkeit zuführt, die der Bewegungsgeschwindigkeit der Windeln (10) während der Herstellung derselben im wesentlichen gleicht, um die elastischen Streifen in praktisch entspannten Zustand zu halten, sowie mit einer zweiten Geschwindigkeit, die kleiner als die Bewegungsgeschwindigkeit der Windeln während der Herstellung derselben ist, um die elastischen Streifen zu strecken.

4. Verfahren nach Anspruch 1 oder 2, dadurch gekennzeichnet, daß man bei dem Schritt des Zuführens der elastischen Streifen (16) diese mit einer vorbestimmten konstanten Geschwindigkeit zuführt, und daß man bei dem Schritt des abwechselnden Streckens und Entspannens vorbestimmter Längen der elastischen Streifen diese während des Zuführens an voneinander beabstandeten Stellen einklemmt, um einen vorbestimmten Wegabstand der elastischen Steifen zwischen den Klemmstellen zu definieren, daß man den Wegabstand der elastischen Streifen zum Strecken von Längen (S) der elastischen Streifen rasch vergrößert und den Wegabstand der elastischen Streifen für eine vorbestimmte Zeitspanne schrittweise weiter vergrößert, um die Streckung der Längen der elastischen Streifen aufrechtzuerhalten, während sie in Position in den Windeln (10) zugeführt werden, und daß man dann den Wegabstand der elastischen Streifen zum Entspannen von Längen (R) der elastischen Streifen rasch verkleinert und den Wegabstand der elastischen Streifen für eine vorbestimmte Zeitspanne schrittweise weiter verkleinert, um die Entspannung der Längen elastischer Streifen aufrechtzuerhalten, während sie in Position in den Windeln zugeführt werden.

5. Vorrichtung zur Durchführung des Verfahrens nach Anspruch 1 zum Anbringen elastischer Streifen (16) in Wegwerf-Windeln (10) mit elastischem Beinabschlu, die geraffte und dehnbare Seitenteile (P) in Schritt-Zonen (C) aufweisen, während der Herstellung und Bewegung der Windeln durch eine Fertigungsmaschine, mit Mitteln (20, 121) zum Zuführen endloser elasticher Streifen (16) in gewünschte Positionen entlang den Längsrängdern der Windeln (10) für eine Klebbefestigung entlang den praktisch ganzen Rändern, gekennzeichnet durch Mittel zum abwechselnden Strecken und Entspannen vorbestimmter Längen elastischer Streifen während des Zuführens derselben, um die gestreckten Längen (S) an den Schritt-Zonen (C) und die mehr entspannten Längen (R) an den äußeren Hüft-Zonen (W) im wesentlichen durchgehend entlang den Längsrändern der Windeln anzubringen.

6. Vorrichtung nach Anspruch 5, dadurch gekennzeichnet, daß die Mittel zum abwechselnden Strecken und Entspannen vorbestimmter Längen der elastischen Streifen (16) wahlweise veränderliche Antriebs-Mittel (125—145) umfassen, die mit den Zuführ-Mitteln (121) für elastische Streifen verbunden sind zum Antrieb der Zuführ-Mittel mit einer ersten Geschwindigkeit, die der Bewegungsgeschwindigkeit der Windeln (10) in der Fertigungs-Maschine im wesentlichen gleicht, um die elastischen Streifen im entspannten Zustand zu halten, und mit einer zweiten Geschwindigkeit, die kleiner als die Bewegungsgeschwindigkeit der Windeln in der Fertigungs-Maschine ist, um die elastische Streifen zu strecken, und daß mit den Antriebs-Mitteln Steuer-Mittel (150, 151) zur abwechselnden Einstellung der Antriebs-Mittel auf die erste und die zweite Zuführgeschwindigkeit der elastischen Streifen betriebsmäßig verbunden sind, um abwechselnd vorbestimmte Längen der elastischen Streifen zu strecken und zu entspannen.

7. Vorrichtung nach Anspruch 6, dadurch gekennzeichnet, daß die wahlweise veränderlichen Antrieb-Mittel erste Antriebsmittel (128—131) umfassen, die mit den Zuführ-Mitteln (121) für elastische Streifen und den Steuermitteln (150, 151) verbunden sind, so daß sie wahlweise zum Antrieb der Zuführ-Mittel für elastischen Streifen mit der ersten Geschwindigkeit betätigbar sind, sowie ferner zweite Antriebsmittel (142—145), die mit den Zuführ-Mitteln für elastische Streifen und den Steuer-Mitteln betriebsmäßig verbunden sind, so daß sie wahlweise zum Antrieb der Zuführ-Mittel für elastische Streifen mit der zweiten Geschwindigkeit betätigbar sind.

8. Vorrichtung nach Anspruch 6 oder 7, dadurch gekennzeichnet, daß die Steuer-Mittel Zeitgeber-Mittel (150) zur wahlweisen Betätigung der ersten und der zweiten Antriebsmittel in Korrelation mit der Bewegungsgeschwindigkeit der Windeln durch die Fertigungsmaschine und der Länge der Längsränder der Schritt-Zonen und der Hüft-Zonen umfassen, an welchen abwechselnde Längen gestreckter und entspannter Abschnitte der elastichen Streifen anzukleben sind.

9. Vorrichtung nach Anspruch 5, dadurch gekennzeichnet, daß die Mittel zum Zuführen der elastischen Streifen (16) Mittel (20) zum Zuführen der elastischen Streifen mit einer vorbestimmten konstanten Geschwindigkeit umfassen, und daß die Mittel zum abwechselnden Strecken und Entspannen vorbestimmter Längen der elastischen Streifen zwei voneinander beabstandete Klemm-Mittel (20, 24 und 26, 27) umfassen, die mit den Zuführ-Mitteln zusammenwirken und die elastischen Streifen während des Zuführens derselben erfassen und eine vorbestimmte Wegstrecke der elastischen Streifen zwischen den Klemm-Mitteln definieren, wobei eines der Klemm-Mittel (26, 27) an der Stelle des Anklebens der elastischen Streifen in den Windeln (10) angeordnet ist, daß bewegliche Mittel (30) zum Ergreifen der elastischen Streifen während ihrer Zufur zwischen den Klemm-Mitteln und zum abwechselnden Bewegen in andere Positionen zum Verändern der Wegstrecke der elastischen Streifen und damit dem abwechselnden Strecken und Entspannen von Längen der elastischen Streifen angeordnet sind, und daß Mittel (32, 33, 35) mit den beweglichen Mitteln (30) verbunden sind zum Bewegen derselben zum schnellen Vergrößern der Wegstrecke der elastischen Streifen zum Strecken von Längen der elastischen Streifen und zum schrittweisen weiteren Vergrößern der Wegstrecke der elastischen Streifen für eine vorbestimmte Zeitspanne zum Aufrechterhalten der Streckung der Längen der elastischen Streifen, während sie in Position in den Windeln zugeführt werden, und dann zum schnellen Verkleinern der Wegstrecke der elastischen Streifen zum Entspannen der elastischen Streifen und zum weiteren schrittweisen Verringern der Wegstrecke der elastischen Streifen während einer vorbestimmten Zeitspanne, um die Entspannung der Längen elastischer Streifen aufrechtzuerhalten, während diese in Position in den Windeln zugeführt werden.

10. Vorrichtung nach Anspruch 9, dadurch gekennzeichnet, daß die Mittel zum abwechselnden Strecken und Entspannen vorbestimmter Längen der elastischen Streifen zeitgesteuerte Steuer-Mittel (40—52) umfassen, die mit den Mitteln (32, 33, 35) zum Bewegen der beweglichen Mittel (30) zur Betätigung dieser Mittel in zeitlicher Korrelation mit der Bewegungsgeschwindigkeit der Windeln durch die Fertigungsmaschine und den Längen der Längsränder der Schritt-Zonen und der Hüft-Zonen verbunden sind, an welchen abwechselnde Längen gestreckter und entspannter Abschnitte der elastischen Bänder anzubringen sind.

11. Vorrichtung nach Anspruch 9 oder 10, dadurch gekennzeichnet, daß die Mittel zum Bewegen der beweglichen Mittel (30) Arbeitszylinder-Mittel (32, 33) umfassen, die mit den beweglichen Mitteln verbunden und für eine schnelle Hin- und Herbewegung zwischen einer eingezogenen und einer ausgefahrenen Stellung zum Bewegen der beweglichen Mittel in die Stellungen zum Strecken und zum Entspannen der elastischen Streifen (16) angebracht sind, sowie Kurbe-Mittel mit einer rotierenden Kurbelscheibe (35) und einem der durch die Arbeitszylinder-Mittel (32, 33) gebildeten Kurbelarm, der mit einem seiner Enden exzentrisch, schwenkbar an der rotierenden Kurbelscheibe angebracht ist und dessen anderes Ende die beweglichen Mittel zur Umformung der Drehbewegung der Kurbelscheibe in eine schrittweise Hin- und Herbewegung der beweglichen Mittel zum Aufrechterhalten der Streckung und der Entspannung der elastischen Streifen trägt.

12. Vorrichtung nach einem der Ansprüche 5 bis 10, gekennzeichnet durch mit den Zuführ-Mitteln (20, 21) zusammenwirkende Mittel (80, 123) zum kontinuierlichen Aufbringen von Klebstoff längs der elastischen Streifen (16) während ihrer Zufuhr mittels der Zuführ-Mittel zum Ankleben der elastischen Streifen in den Windeln.

13. Wegwerf-Windel (10) mit elastischem Beinabschluß, hergestellt nach dem Verfahren gemäß einem der Ansprüche 1 bis 4 oder mittels der Vorrichtung gemäß einem der Ansprüche 5 bis 12.

## Revendications

1. Procédé pour fixer des bandes élastiques (16) dans les bords longitudinaux de couches culottes élastiques (10) à jeter après usage, ayant une zone centrale (C) d'adaptation à l'entrejambe et des zones externes (W) d'adaptation à la taille, les bandes élastiques (16) s'étendant le long de la zone centrale (C) entre les zones de taille (W) pour former des parties latérales (P) froncées et extensibles dans la zone centrale de la couche pendant la fabrication de celle-ci, le procédé comprenant les étapes d'alimenter en continu des bandes élastiques (16) à partir d'un magasin, dans la position désirée en contact dans les bords longitudinaux de la couche (10), tout en y effectuant la fixation par adhésif sensiblement sur toute la longueur de chaque bord, le procédé étant caractérisé par l'étirement et le relâchement en alternance de longueurs prédéterminées des bandes élastiques pendant l'étape d'alimentation, cependant que l'on fait adhérer les longueurs (S) en extension des bandes élastiques sur les zones d'entrejambe (C) et les longueurs (R) plus relâchées des bandes élastiques aux zones de taille de façon sensiblement continue sur tous les bords longitudinaux de la couche, de façon que les longueurs (S) en extension des bandes élastiques se contractent et forment des parties latérales (P) froncées et extensibles dans la zone d'entrejambe de la couche.

2. Procédé selon la revendication 1, caractérisé par l'étape d'appliquer de l'adhésif de façon sensiblement continue sur la longueur des bandes élastiques (16).

3. Procédé selon une des revendications 1 ou 2, caractérisé en ce que l'étape d'étirement et de relâchement alternatifs de longueurs prédéterminées de bandes élastiques (16) comprend l'alimentation de bandes élastiques à une première vitesse sensiblement égale à la vitesse du mouvement de la couche (10) pendant la fabrication de la couche pour maintenir les bandes élastiques dans une condition sensiblement relâchée, et à une seconde vitesse inférieure à la vitesse du mouvement de la couche pendant la fabrication de la couche pour étirer les bandes élastiques.

4. Procédé selon une des revendications 1 ou 2, caractérisé en ce que l'étape d'alimenter les bandes élastiques (16) comprend l'alimentation des bandes à un débit prédéterminé constant, et l'étape d'étirer et de relâcher alternativement des bandes élastiques comprend le pincement des bandes élastiques pendant leur alimentation à des points distants les uns des autres pour définir une distance de déplacement prédéterminée des bandes élastiques entre les points de pincement, l'accroissement rapide de la distance de déplacement des bandes élastiques pour étirer des longueurs (S) de bandes élastiques, et par paliers continuer à accroître la distance de déplacement des bandes élastiques pendant une période de temps prédéterminée pour maintenir l'extension des longueurs des bandes élastiques pendant qu'elles sont alimentées en place dans les couches (10), et ensuite la décroissance rapide de la distance de déplacement des bandes élastiques pour relâcher des longueurs (R) des bandes élastiques et décroître ensuite par paliers la distance de déplacement des bandes élastiques pendant une période de temps prédéterminée pour maintenir le relâchement des longueurs de bandes élastiques pendant qu'elles sont alimentées en place dans la couche.

5. Appareil pour la mise en oeuvre du procédé revendiqué dans la revendication 1, pour attacher des bandes élastiques dans des couches culottes élastiques (10) à jeter après usage, ayant des parties latérales (P) froncées et extensibles dans la zone (C) de l'entrejambe, pendant la fabrication et le déplacement des couches dans une machine d'assemblage, comportant un moyen (20, 21) pour alimenter en continu des bandes élastiques (16) aux emplacements désirés le long des bords longitudinaux des couches (10) pour la fixation par adhésif sensiblement sur toute la longueur des bords, appareil caractérisé en ce qu'il comporte un moyen pour étirer et relâcher alternativement des longueurs prédéterminées des bandes élastiques pendant leur alimentation pour attacher les longueurs étirées (S) sur les zones (C) de l'entrejambe et les longueurs plus relâchées (R) aux zones externes (W) de la taille, de façon sensiblement continue le long des bords longitudinaux des couches.

6. Appareil selon la revendication 5, caractérisé en ce que le moyen pour étirer et relâcher alternativement des longueurs prédéterminées de bandes élastiques (16) comprend des moyens d'entrainement sélectivement variables (125, 145) connectés au moyen d'alimentation (121) en bande élastique, pour entrainer ce moyen d'alimentation à une première vitesse sensiblement égale à la vitesse de déplacement des couches (10) dans la machine d'assemblage pour maintenir les bandes élastiques en condition relâchée, et à une seconde vitesse inférieure à la vitesse de déplacement des couches dans la machine d'assemblage pour étirer les bandes élastiques, et un moyen de contrôle (150, 151) connecté de façon opérante aux moyens d'entraînement pour actionner alternativement les moyens d'entraînement pour la première et la seconde vitesse d'alimentation des bandes élastiques pour étirer et relâcher alternativement des longueurs prédéterminées des bandes élastiques.

7. Appareil selon la revendication 6, caractérisé en ce que les moyens d'entraînement sélectivement variables comportent un premier moyen d'entraînement (128, 131) connecté au moyen d'alimentation (121) en bande élastique et au moyen de contrôle (150, 151) pour être actionné sélectivement pour entraîner le moyen d'alimentation en bande élastique à la première vitesse et un second moyen d'entraînement (142, 145) connecté de façon opérante au moyen d'alimentation en bande élastique et au moyen de contrôle pour être actionné sélectivement pour entraîner le moyen d'alimentation en bande élastique à la seconde vitesse.

8. Appareil selon une des revendications 6 ou 7, caractérisé en ce que le moyen de contrôle comporte une base de temps (150) pour actionner alternativement le premier et le second moyen d'entrainement en corrélation avec la vitesse de déplacement des couches à travers la machine d'assemblage et les longueurs des bords longitudinaux de la zone d'entrejambe et des zones de taille auxquelles doivent adhérer des longueurs alternées de portions étirées et relâchées des bandes élastiques.

9. Appareil selon la revendication 5, caractérisé en ce que le moyen pour alimenter la bande élastique (16) comprend un organe (20) pour alimenter la bande élastique à un débit prédéterminé constant, et le moyen pour étirer et relâcher alternativement des longueurs prédéterminées des bandes élastiques comprend deux organes de pincement (20, 24 et 26, 27) écartés l'un de l'autre coopérant avec le moyen d'alimentation et attaquant les bandes élastiques pendant leur alimentation, en définissant une distance de déplacement prédéterminée des bandes élastiques entre les points de pincement, un des organes de pincement (26, 27) étant placé au point de fixation par adhérence de la bande élastique dans la couche (10): un organe mobile (30) placé pour attaquer les bandes élastiques lorsqu'elles sont alimentées entre les organes de pincement et pour déplacer alternativement à d'autres positions pour faire varier la distance de déplacement des bandes élastiques pour étirer et relâcher alternativement des longueurs de bandes élastiques, et des moyens (32, 33, 35) connectés audit organe mobile (30) pour déplacer celui-ci en vue d'un accroissement rapide de la distance de déplacement des bandes élastiques pour étirer des longueurs de bande élastique et accroître en outre par paliers la distance de déplacement des bandes élastiques pendant une période de temps prédéterminée pour maintenir l'extension des longueurs des bandes élastiques quand elles sont alimentées en place dans les couches, et ensuite faire décroître rapidement la distance de déplacement des bandes élastiques pour relâcher des longueurs de bandes élastiques et faire décroître ensuite par paliers la distance de déplacement des bandes élastiques pendant une période de temps prédéterminée pour maintenir la relaxation des longueurs de bandes élastiques pendant quelles sont alimentées en place dans les couches.

10. Appareil selon la revendication 9, caractérisé en ce que le moyen pour relâcher et étirer alternativement des longueurs prédéterminées de bandes élastiques comprend des moyens de contrôle temporisé (40, 52) connectés aux moyens (32, 33, 35) pour déplacer l'organe mobile (30) pour actionner ces moyens en corrélation temporisée avec la vitesse de déplacement des couches à travers la machine d'assemblage et les longueurs des bords longitudinaux des zones d'entrejambe et des zones de la taille auxquelles doivent adhérer des longueurs alternées de portions étirées et relâchées de bandes élastiques.

11. Appareil selon une des revendications 9 ou 10, caractérisé en ce que le moyen pour déplacer l'organe mobile (30) comprend un piston et un cylindre (32, 33) connectés à cet organe mobile et montés pour un mouvement rapide vers une position contractée et à partir de celle-ci, et vers une position étirée et à partir de celle-ci pour déplacer l'organe mobile aux positions pour étirer et relâcher les bandes élastiques (16), et un système de manivelle comportant une roue d'entraînement de manivelle (35) et un bras de manivelle formé par lesdits piston et cylindre (32, 33), dont une extrémité est articulée excentriquement sur ladite roue (35) et l'autre portant ledit organe mobile (30) pour transformer le mouvement rotatif de la roue en mouvements alternatifs pas à pas dudit organe mobile pour maintenir l'étirement et le relâchement des bandes élastiques.

12. Appareil selon une des revendications 5 à 10, caractérisé en ce qu'il comporte des moyens (28, 123) coopérant avec le moyen d'alimentation (20, 121) pour appliquer l'adhésif de façon continue le long des bandes élastiques (16) quand elles sont alimentées par ledit moyen d'alimentation pour les fixer par adhérence des bandes élastiques dans les couches.

13. Couche-culotte élastique (10) à jeter après usage produite par le procédé selon une des revendications 1 à 4 ou au moyen de l'appareil selon une des revendications 5 à 12.

Fig-1

Fig-2

Fig-3

Fig-4

Fig-5

FIG-6

FIG-7

2

FIG-8

FIG-9

FIG-10

23

52

51

11

16

26

20 24
21
20
24
17
16
28
32
28"
45 46
27
30
29

33

35

54

53

16

FIG-11

20 30
24 21
28
20
28
24
29
26
27
30
16

40

43
44

41
42

45
56
32
46
53 33
35
52
50
49 51
47
48

FIG-12

4

FIG-14

FIG-13

0 027 303

0 027 303

FIG-16

FIG-17

FIG-15

FIG-18

FIG-19